# EUROPÄISCHE PATENTSCHRIFT

(11) **EP 3 503 937 B1**
(45) Veröffentlichungstag und Bekanntmachung des Hinweises auf die Patenterteilung: **28.07.2021**
(21) Anmeldenummer: 17751085.6
(22) Anmeldetag: 09.08.2017
(51) Int. Cl.: A61M 1/00

(54) **VORRICHTUNG ZUR UNTERDRUCKBEHANDLUNG VON WUNDEN AM MENSCHLICHEN KÖRPER**
DEVICE FOR THE VACUUM TREATMENT OF WOUNDS ON THE HUMAN BODY
DISPOSITIF DE TRAITEMENT PAR PRESSION NÉGATIVE DE PLAIES AFFECTANT LE CORPS D'UNE PERSONNE

(30) Priorität: 25.08.2016 DE 102016115834
(43) Veröffentlichungstag der Anmeldung: 03.07.2019
(73) Patentinhaber: Paul Hartmann AG, 89522 Heidenheim (DE)
(72) Erfinder: HOFSTETTER, Juergen, 52349 Düren (DE); ECKSTEIN, Axel, 89522 Heidenheim (DE); BEYRLE, Karina, 89075 Ulm (DE); MOTTSCHELLER, Markus, 90489 Nuernberg (DE); KLEIN, Peter, 71254 Ditzingen (DE); MURGULESCU, Mihai, 90411 Nuernberg (DE)
(74) Vertreter: DREISS Patentanwälte PartG mbB
(86) Internationale Anmeldenummer: PCT/EP2017/070205
(87) Internationale Veröffentlichungsnummer: WO 2018/036822

(56) Entgegenhaltungen:
- WO-A1-2009/004371
- US-A1- 2009 240 218
- US-A1- 2012 209 228
- US-A1- 2013 267 918
- US-A1- 2014 276 498
- US-A1- 2015 025 482

## Beschreibung

Die Erfindung betrifft eine Vorrichtung zur Bereitstellung von Unterdruck zur Unterdruckbehandlung von Wunden am menschlichen Körper, mit einer Unterdruck erzeugenden Einrichtung und einem Behälter zur Aufnahme von Flüssigkeiten in einem Inneren des Behälters, insbesondere von aus einer Wunde abgesaugten Wundsekreten, mit einem Anschluss für eine zum Körper führende Saugleitung, wobei die Unterdruck erzeugende Einrichtung in einem ersten Gehäuseteil der Vorrichtung angeordnet ist und der Behälter von einem zweiten Gehäuseteil der Vorrichtung gebildet ist, wobei die Gehäuseteile lösbar gegeneinander fixierbar sind, und die Vorrichtung ein gurtartiges Haltemittel aufweist, so dass sie am Körper des Benutzers tragbar und mitführbar ist, wobei die Vorrichtung eine in einer mobilen Trageposition dem Körper des Benutzers zugewandte Rückseite und eine dieser gegenüberliegende vom Körper des Benutzers abgewandte und eine Sichtseite der Vorrichtung bildende Vorderseite und eine diese verbindende Umfangsseite umfasst.

Wenn vorstehend von einer tragbaren und mitführbaren Vorrichtung die Rede ist, so bedeutet dies, dass der Patient die Vorrichtung am Körper mitführen kann, so dass er mobil ist und seine Wunde dennoch dauerhaft, d.h. ohne Unterbrechung, therapiert werden kann. Die tragbare Vorrichtung kann dabei mittels des gurtartigen Haltemittels am Körper des Patienten gehalten und mitgeführt werden. Eine tragbare Vorrichtung der hier in Rede stehenden Art kann aber auch im stationären Betrieb, also losgelöst vom Körper des Patienten, eingesetzt werden; sie kann solchenfalls beispielsweise an einem Pflegebett befestigt oder neben dem Pflegebett abgestellt werden.

Unter einer Trageposition wird im Sinne der vorliegenden Erfindung eine Trageposition der Vorrichtung am Körper eines aufrecht stehenden Benutzers verstanden, wobei die Vorrichtung insbesondere taschenartig wie eine Tasche über die Schulter des Benutzers gehängt getragen werden kann. Dies bedeutet aber nicht, dass die Vorrichtung nicht auch im Sitzen oder Liegen am Körper des Benutzers gehalten sein kann. Auch dann liegt eine Tragesituation vor.

Derartige Vorrichtungen zur Unterdruckwundbehandlung sind bereits mehrfach aus dem Stand der Technik bekannt. Aus der US 2009/0240218 A1 ist eine Vorrichtung zur Bereitstellung von Unterdruck zur Unterdruckbehandlung am menschlichen Körper bekannt, die in einem beutelartigen Haltemittel am Körper eines Benutzers mitführbar ist. Ferner ist aus der US 2015/0025482 A1 eine Vorrichtung zur Bereitstellung von Unterdruck zur Unterdruckbehandlung am menschlichen Körper bekannt, die Befestigungsmittel für einen Riemen aufweist um die Vorrichtung am Körper eines Benutzers mitzuführen.

Bei derartigen Vorrichtungen zur Unterdruckbehandlung von Wunden kommuniziert eine Unterdruck erzeugende Einrichtung über eine Saugleitung mit der Wunde oder der Wundumgebung, wobei ein luftundurchlässiges Abdeckmaterial zum luftdichten Verschließen der Wunde und der Wundumgebung vorgesehen ist, so dass ein Unterdruck im Wundraum herstellbar ist und Flüssigkeiten aus dem Wundraum in den genannten Behälter absaugbar sind.

Der Begriff eines Unterdrucks bezeichnet im Zusammenhang mit der vorliegenden Erfindung einen gegenüber dem Umgebungsluftdruck (atmosphärischer Luftdruck) erniedrigten Luftdruck insbesondere innerhalb eines Wundverbands. Das Abdeckmaterial eines Wundverbands zum luftdichten Verschließen eines Wundraums muss daher so ausgebildet sein, dass es der sich einstellenden Druckdifferenz standzuhalten vermag, damit der Unterdruck im Wundraum überhaupt angelegt und aufrechterhalten werden kann.

Im Bereich der Unterdrucktherapie in der Wundbehandlung wird der Unterdruck quantitativ als Druckdifferenz zwischen dem Umgebungsluftdruck und dem unterhalb des Abdeckmaterials angelegten Luftdruck angegeben. Typischerweise beträgt diese Druckdifferenz im Bereich der Unterdrucktherapie höchstens 250 mm Hg (mm Quecksilbersäule) (1 mm Hg = 1 Torr. = 133,322 Pa). Dieser Unterdruckbereich bis höchstens 250 mm Hg hat sich als für die Wundheilung geeignet erwiesen. Ein bevorzugter Unterdruckbereich liegt zwischen 10 und 150 mm Hg.

Der unter Verwendung der Vorrichtung an die Wunde angelegte Unterdruck kann bei einer typischen Unterdruckbehandlung entweder zeitlich im Wesentlichen konstant gehalten werden, oder er kann zeitlich verändert, insbesondere zyklisch verändert werden, was über eine entsprechend ausgebildete und programmierte Steuervorrichtung bei der Unterdruck erzeugenden Einrichtung, insbesondere in Abhängigkeit weiterer Parameter, realisiert werden kann.

Zum Anlegen von Unterdruck und vorzugsweise auch zum Absaugen von Körperflüssigkeiten ist eine vorzugsweise flexible Saugleitung, beispielsweise in Form eines Drainageschlauchs, vorgesehen, der einenends über einen sogenannten Port im Bereich des Wundabdeckmaterials mit der Wundumgebung oder dem Wundraum und anderenends mit dem eingangs erwähnten Behälter zur Aufnahme von Körperflüssigkeiten bzw. mit der Unterdruck erzeugenden Einrichtung kommuniziert.

Generell sind eine einfache und benutzerfreundliche Handhabbarkeit sowie eine diskrete Erscheinungsform der medizinischen Vorrichtung wünschenswert.

Der vorliegenden Erfindung liegt daher die Aufgabe zugrunde, eine Vorrichtung der vorausgehend beschriebenen Art dahingehend zu verbessern, dass sie auf kostengünstige Weise eine benutzerfreundliche Handhabbarkeit mit einem diskreten alltagstauglichen Erscheinungsbild vereint.

Diese Aufgabe wird bei einer Vorrichtung der genannten Art erfindungsgemäß dadurch gelöst, dass das Haltemittel derart an der Vorrichtung lösbar fixierbar ist, dass das Haltemittel in einer Betriebsstellung einen transparenten Bereich einer Wandung des zweiten Gehäuseteils abdeckt, durch den eine Einsichtnahme in das Innere des Behälters möglich ist, wenn sich das Haltemittel nicht in der Betriebsstellung befindet.

Eine Einsehbarkeit in das Innere des Behälters ermöglicht dem Benutzer eine schnelle und unkomplizierte visuelle Beurteilung der im Behälter vorhandenen Flüssigkeiten. Die Wandung des zweiten Gehäuseteils umfasst dafür einen hinreichend durchsichtigen Bereich, insbesondere ein Sichtfenster, durch den eine Einsichtnahme möglich ist, für den Fall, dass sich das Haltemittel nicht in der Betriebsstellung befindet. Befindet sich das Haltemittel hingegen in der Betriebsstellung, so deckt es den transparenten Bereich in der Wandung des zweiten Gehäuseteils gewolltermaßen ab, um eine unerwünschte Einsichtnahme in das Innere des Behälters zu verhindern. Das Haltemittel verwirklicht auf diese Weise zwei wichtige Funktionen. Es ermöglicht die Tragbarkeit der Vorrichtung und erfüllt eine Abdeckfunktion für den transparenten Bereich.

Das gurtartige Haltemittel ist vorzugsweise ein flexibles streifenförmiges, und vorzugsweise längenverstellbares Haltemittel, insbesondere aus Nylongewebe oder aus einem anderen an sich beliebigen geeigneten biegsamen Material. Unter einem flexiblen Haltemittel ist dabei ein Haltemittel zu verstehen, welches nicht formstabil sondern biegsam ist, welches sich also beim Tragen durch einen Patienten dessen Körperform anzupassen vermag. Ferner ist es bevorzugt, wenn das Haltemittel eine, insbesondere gepolsterte, Schulterauflage umfasst, um den Tragekomfort der Vorrichtung weiter zu verbessern.

Erfindungsgemäß ist der transparente Bereich der Wandung des zweiten Gehäuseteils in der Umfangsseite der Vorrichtung ausgebildet. Die Umfangsseite der Vorrichtung verbindet die in der mobilen Trageposition dem Körper des Benutzers zugewandte Rückseite mit der dieser gegenüberliegenden vom Körper des Benutzers abgewandten und eine Sichtseite der Vorrichtung bildenden Vorderseite. Der transparente Bereich kann an beliebiger Stelle in der Umfangsseite ausgebildet sein. Vorzugsweise ist der transparente Bereich in einem in der mobilen Trageposition seitlich und/oder unten liegenden Bereich der Umfangsseite ausgebildet.

In weitergehender Ausbildung der Erfindung erweist es sich als vorteilhaft, wenn das Haltemittel in der Betriebsstellung die Vorrichtung im Bereich der Umfangsseite umfänglich zumindest teilweise umgibt, so dass die Umfangsseite der Vorrichtung zumindest teilweise von dem Haltemittel bedeckt ist. Vorzugsweise umläuft das Haltemittel die Vorrichtung derart, dass in der mobilen Trageposition betrachtet ein unten liegender Bereich der Umfangsseite und seitlich liegende Bereiche der Umfangsseite von dem Haltemittel zumindest teilweise, vorzugsweise vollständig, bedeckt werden.

Vorzugsweise weist die Vorrichtung Führungselemente für das Haltemittel auf. Die Führungselemente können insbesondere von abgewinkelten Stegen und/oder Laschen gebildet sein.

In weitergehender Ausbildung der Erfindung erweist es sich als vorteilhaft, wenn ein Führungselement entlang der Umfangsseite erstreckt ist. Hierdurch ist es möglich, dass das Haltemittel die Vorrichtung in Umfangsrichtung geführt umläuft.

Vorzugsweise ist das Führungselement durch eine vorspringende die Vorder- und/oder Rückseite bildende Deckplatte gebildet. Die Umfangsseite der Vorrichtung ist gegenüber der Deckplatte dann zurückgesetzt, so dass auf diese Weise ein entlang der Umfangsseite der Vorrichtung schienenartig erstrecktes Führungselement gebildet ist.

In weitergehender Ausbildung der Erfindung erweist es sich als vorteilhaft, wenn die Vorrichtung Befestigungselemente, insbesondere Haken-/Schlaufen-Befestigungselemente, aufweist, an denen das Haltemittel, lösbar fixierbar ist. Um Einblick in das Innere des Behälters zu gewähren, kann die Fixierung des Haltemittels gelöst werden.

In weitergehender Ausbildung der Erfindung erweist es sich als vorteilhaft, wenn die Gehäuseteile über eine Schiebesitzführung aneinander gefügt werden.

Es erweist sich als vorteilhaft, wenn wenigstens ein Gehäuseteil in einer Seitenansicht im Wesentlichen L-förmig ausgebildet oder ergänzt ist, und die L-Form eine Schiebesitzführung bildet. Im Sinne der vorliegenden Erfindung ist mit einer L-Form gemeint, dass zwei Schenkel oder zwei Teile eines Gehäuseteils unter einem Winkel von etwa 85° bis 95°, vorzugsweise 90° zusammenlaufen oder zueinander erstreckt sind, wobei die beiden Schenkel oder Teile des Gehäuseteils eine gleich große oder eine unterschiedlich große Erstreckung aufweisen können.

Der erste und der zweite Gehäuseteil sind, insbesondere zum Wechseln des Behälters, welcher durch den zweiten Gehäuseteil gebildet wird, lösbar aneinander fixierbar. Zum Aneinanderfügen der beiden Gehäuseteile erweist es sich als vorteilhaft, wenn ein Gehäuseteil geführt auf den anderen Gehäuseteil aufschiebbar ist.

Im Hinblick auf ein benutzerfreundliches Fügen der beiden Gehäuseteile erweist es sich als vorteilhaft, wenn auf einer Innenseite einer Schenkelplatte des L-förmigen Gehäuseteils wenigstens ein erstes Schiebeführungsmittel ausgebildet ist, und der andere Gehäuseteil ein zu dem ersten Schiebeführungsmittel komplementäres zweites Schiebeführungsmittel umfasst, so dass der andere Gehäuseteil auf die Schenkelplatte des L-förmigen Gehäuseteils translatorisch geführt aufschiebbar ist, wobei die Schiebeführungsmittel ineinander eingreifen. Werden die ersten und zweiten komplementären Schiebeführungsmittel miteinander in Eingriff gebracht, können die beiden Gehäuseteile durch eine translatorische Schiebebewegung geführt in ihre bestimmungsgemäße Position gebracht werden.

Vorzugsweise umfassen die Schiebesitzführung oder das erste und/oder das zweite Schiebeführungsmittel wenigstens eine(n) Führungsschiene, -rippe, -steg und/oder wenigstens eine dazu komplementäre Führungsnut, -aufnahme oder - vertiefung.

Es erweist sich als vorteilhaft, wenn in einem bestimmungsgemäß gefügten Zustand der zweite Gehäuseteil mit dem ersten Gehäuseteil auf Stoß liegt. Hierdurch wird vermieden, dass zwischen den beiden Gehäuseteilen insbesondere Schmutz aufnehmende Fugen, Ritzen oder Spalten gebildet werden.

Vorzugsweise ist der erste Gehäuseteil in der Seitenansicht im Wesentlichen L-förmig ausgebildet und der zweite Gehäuseteil weist ein den ersten Gehäuseteil komplementär ergänzendes Profil auf, so dass die Vorrichtung im Wesentlichen eine Scheibenform aufweist, wenn sich die Gehäuseteile in dem bestimmungsgemäß gefügten Zustand befinden. Die scheibenförmige Ausbildung der Vorrichtung bedeutet im Sinne der vorliegenden Erfindung, dass die bestimmungsgemäß zusammengefügten Gehäuseteile, wenn sie sich in einer Tragesituation oder Trageposition am Körper eines Benutzers befinden, jeweils eine Breite und Höhe aufweisen, die gegenüber einer Tiefe in horizontaler Richtung betrachtet größer sind. Vorzugsweise erhält die Vorrichtung durch die Scheibenform der bestimmungsgemäß zusammengefügten Gehäuseteile in Kombination mit dem gurtartigen Haltemittel ein taschenartiges Erscheinungsbild.

Vorzugsweise ist der erste Gehäuseteil derart ausgebildet, dass die Unterdruck erzeugende Einrichtung in einem Teil des ersten Gehäuseteils angeordnet ist und sich ausgehend von diesem Teil eine Wandung des ersten Gehäuseteils plattenartig vorspringend erstreckt, wobei diese plattenartig vorspringende Wandung die oben erwähnte Schenkelplatte bilden kann und der erste Gehäuseteil auf diese Weise ein in der Seitenansicht betrachtet L-förmiges Profil aufweist. Vorzugsweise ist der zweite Gehäuseteil komplementär zu dem L-förmigen Profil, insbesondere quader- oder scheibenförmig ausgebildet. Die beiden Gehäuseteile werden bestimmungsgemäß zusammengefügt, indem der zweite Gehäuseteil auf die plattenartig vorspringende Schenkelplatte des ersten Gehäuseteils aufgeschoben wird.

In weiterer Ausbildung der Erfindung weist die Vorrichtung wenigstens ein schnappendes, rastendes oder in sonstiger Weise hintergreifend wirkendes Verriegelungsmittel auf, mittels dessen der zweite Gehäuseteil gegen den ersten Gehäuseteil lösbar fixierbar ist. Hierdurch werden die beiden Gehäuseteile in ihrem bestimmungsgemäß zusammengefügten Zustand gehalten. Zum Lösen der zusammengefügten Gehäuseteile ist das Verriegelungsmittel freigebend betätigbar.

Vorzugsweise umfasst das wenigstens eine Verriegelungsmittel eine rückfedernd auslenkbare Verriegelungslasche und ein dazu komplementäres hintergreifbares Element. Die Verriegelungslasche kann insbesondere derart ausgebildet sein, dass sie beim translatorischen Aufschieben des einen, insbesondere zweiten Gehäuseteils auf den anderen, insbesondere ersten Gehäuseteil, vorzugsweise selbsttätig ausgelenkt wird und anschließend dann in eine die Gehäuseteile gegeneinander verriegelnde Stellung einrastet.

In weitergehender Ausbildung der Erfindung erweist es sich als vorteilhaft, wenn in einem Endbereich der ersten und zweiten Schiebeführungsmittel des ersten und zweiten Gehäuseteils zusammenwirkende Verriegelungsmittel ausgebildet sind. Die Verriegelungsmittel können insbesondere schnappende, rastende oder in sonstiger Weise hintergreifend wirkende Verriegelungsmittel sein.

Nach einem weiteren Erfindungsgedanken von wesentlicher Bedeutung erweist es sich als vorteilhaft, dass die Sichtseite der Vorrichtung zumindest teilweise durch eine Schenkelplatte eines L-förmigen Gehäuseteils und die Rückseite zumindest teilweise durch eine hintere Wand des anderen Gehäuseteils gebildet wird.

Es erweist sich als besonders vorteilhaft, dass die Sichtseite und/oder die Rückseite der Vorrichtung aus einem nicht transparenten Material gefertigt ist, und im bestimmungsgemäß zusammengefügten Zustand eine Schenkelplatte des ersten Gehäuseteils den zweiten Gehäuseteil in frontaler Blickrichtung auf die körperabgewandte Sichtseite der Vorrichtung zu wenigstens 90% oder vorzugsweise vollständig verdeckt. Hierdurch kann die gesamte Sichtseite der Vorrichtung im Wesentlichen von einer durchgehenden Oberfläche, insbesondere von der Schenkelplatte, gebildet werden, so dass insbesondere keine Schmutz aufnehmenden Fugen entstehen und ein einheitliches Erscheinungsbild resultiert. Ferner ist ein Einblick in das Innere des den Behälter bildenden zweiten Gehäuseteils für Dritte weitestgehend unmöglich, wenn der zweite Gehäuseteil in der Trageposition oder Tragesituation körperzugewandt orientiert ist und in frontaler Blickrichtung auf die körperabgewandte Sichtseite vorzugsweise vollständig von der Schenkelplatte des ersten Gehäuseteils in dieser Blickrichtung verdeckt ist. In Kombination mit dem gurtartigen Haltemittel, welches vorzugsweise die Umfangsseite der Vorrichtung umläuft, könnte eine unmittelbare Einsichtnahme in das Innere des Behälters auch dann verhindert werden, wenn der zweite Gehäuseteil oder der Behälter an mehreren Seiten oder insbesondere ganz durchsichtig ausgebildet ist.

Gemäß einem weiteren Erfindungsgedanken erweist es sich als vorteilhaft, dass der erste und der zweite Gehäuseteil der Vorrichtung im bestimmungsgemäß zusammengesetzten Zustand in der mobilen Trageposition betrachtet vertikal übereinander angeordnet sind, so dass zwischen den Gehäuseteilen eine im Wesentlichen horizontale Trennebene ausgebildet ist, wobei ein oberer Teil der Vorrichtung von dem ersten, die Unterdruck erzeugende Einrichtung umfassenden Gehäuseteil und ein unterer Teil der Vorrichtung von dem zweiten den Behälter bildenden Gehäuseteil gebildet wird. Wenn vorausgehend von einer Trennebene die Rede ist, so bedeutet dies aber nicht zwangsläufig, dass die Gehäuseteile über eine exakt ebene Fläche gegeneinander anliegen. Vielmehr können im Bereich der Trennebene Anschlüsse zur Unterdruckkommunikation oder sonstige Passungen, insbesondere eine zusätzliche Schenkelplatte als Schiebesitzführung, zwischen den beiden Gehäuseteilen ausgebildet sein. Die Gehäuseteile liegen im bestimmungsgemäß gefügten Zustand über die genannte im Wesentlichen horizontal orientierte Trennebene übereinander und gegeneinander an, wenn die Vorrichtung in der Trageposition am Körper eines aufrecht stehenden Menschen getragen wird. Auch wenn einer der Gehäuseteile L-förmig ausgebildet ist, so befindet sich der die Unterdruck erzeugende Einrichtung umfassende Bereich des ersten Gehäuseteils oberhalb des Behälters zur Aufnahme von Flüssigkeiten.

Es erweist sich als vorteilhaft, wenn auf der Rückseite der Vorrichtung Aufnahmemittel, insbesondere eine Aufnahmevertiefung, für die zum Körper führende Saugleitung ausgebildet ist.

Vorzugsweise weist die Vorrichtung in einem in der Trageposition oberen Teil der Umfangsseite, welcher von dem in der Betriebsstellung befindlichen Haltemittel nicht überdeckt wird, Bedien- und Anzeigeelemente auf. Hierdurch ist auch der Zugriff und die Bedienung der Vorrichtung durch den Benutzer der Vorrichtung selbst möglich, indem die Bedienkomponenten und Anzeigekomponenten dann körperabgewandt und vorzugsweise von oben einsehbar angeordnet sind.

Neben der Unterdruckwundbehandlung sind auch andere Anwendungen der hier in Rede stehenden Vorrichtung zur Bereitstellung von Unterdruck für medizinische Anwendungen denkbar, insbesondere die Absaugung an sich beliebiger Körperflüssigkeiten, im Bereich der medizinischen Versorgung, insbesondere Inkontinentenversorgung, der Versorgung von Stoma-Patienten oder im Bereich der Absaugung von Wundsekreten, gegebenenfalls unter Verwendung von Spülflüssigkeiten, auch ohne Anlegen eines Unterdrucks über wesentliche Zeiträume.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Vorrichtung. Für die Merkmale der Patentansprüche wird jeweils separat und losgelöst von einer Rückbeziehung der Ansprüche in beliebiger Kombination Schutz in Anspruch genommen. In der Zeichnung zeigt:
- Fig. 1a bis 1c: verschiedene Ansichten einer bevorzugten Ausführungsform der erfindungsgemäßen am Körper eines Benutzers tragbaren Vorrichtung zur Bereitstellung von Unterdruck für medizinische Anwendungen, und
- Fig. 2a bis 2g: verschiedene Ansichten eines eine Unterdruck erzeugende Einrichtung und Steuerungskomponenten umfassenden ersten Gehäuseteils und eines einen Behälter zur Aufnahme von Körperflüssigkeiten bildenden zweiten Gehäuseteils der Vorrichtung nach Figuren 1a bis 1c.

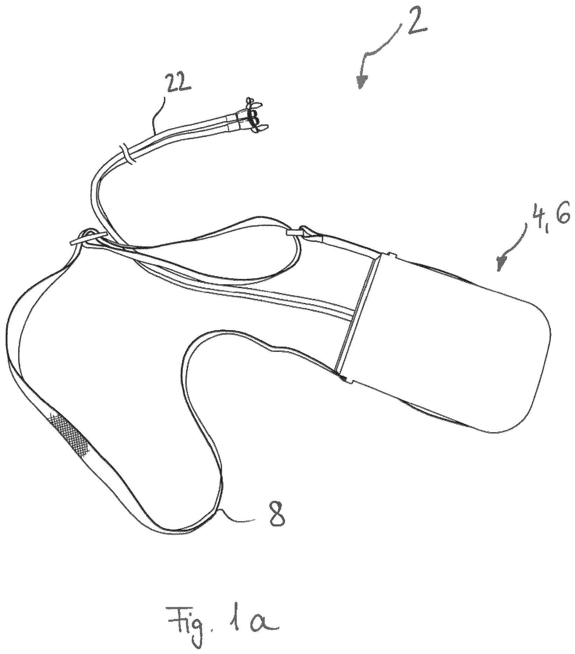

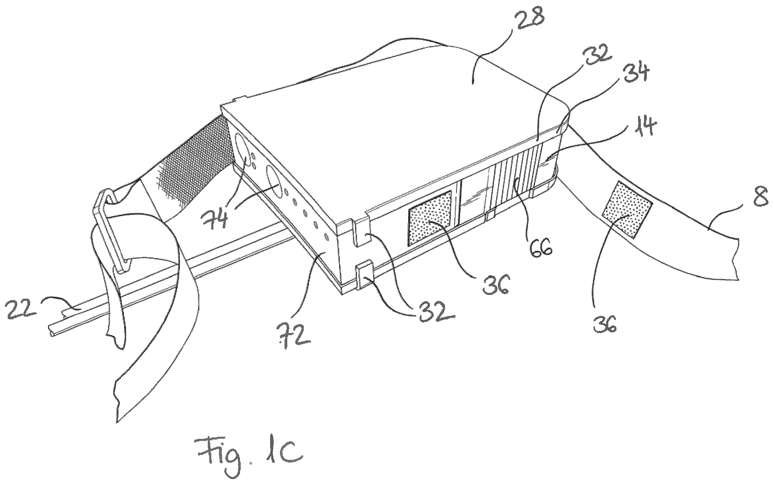

Die Figuren 1a bis c zeigen eine erste Ausführungsform einer erfindungsgemäßen tragbaren Vorrichtung 2 zur Bereitstellung von Unterdruck für medizinische Anwendungen. Die Vorrichtung 2 umfasst zwei lösbar gegeneinander fixierbare Gehäuseteile 4, 6, sowie ein gurtartiges Haltemittel 8, so dass die Vorrichtung 2 am Körper eines Benutzers tragbar und mitführbar ist. Das Haltemittel 8 ist vorzugsweise ein flexibles streifenförmiges, riemenartiges längenverstellbares Haltemittel 8, insbesondere aus Nylongewebe oder einem beliebigen anderen Material. Ferner ist es bevorzugt, wenn das Haltemittel eine, insbesondere gepolsterte, Schulterauflage (nicht dargestellt) umfasst. Hierdurch wird der Tragekomfort der Vorrichtung weiter verbessert.

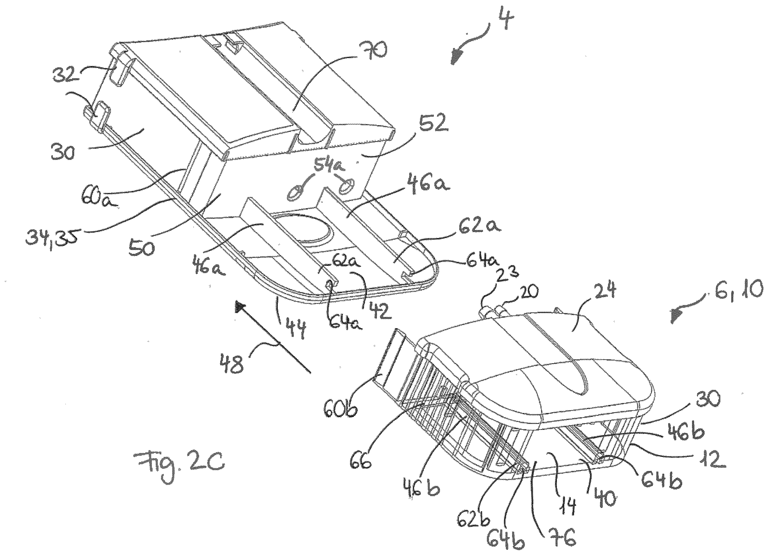

Fig. 2c zeigt die Gehäuseteile 4, 6 in einer separat voneinander angeordneten Position. In dem ersten Gehäuseteil 4 ist eine Unterdruck erzeugende Einrichtung vorzugsweise in Form einer Luftpumpe sowie elektrische und elektronische Steuerkomponenten für die Vorrichtung 2 einschließlich Batterien oder vorzugsweise wiederaufladbarer Akkus aufgenommen. Der zweite Gehäuseteil 6 bildet im bevorzugten Fall zugleich einen Behälter 10 zur Aufnahme von Körperflüssigkeiten, insbesondere zur Aufnahme von aus einer Wunde abgesaugten Wundsekreten.

In einer Wandung 12 des zweiten Gehäuseteils 6 ist ein transparenter Bereich 14 ausgebildet, durch den eine Einsichtnahme in das Innere 16 des Behälters 10 möglich ist, um insbesondere den Füllstand im Behälter 10 zu überprüfen. Vorzugsweise ist der gesamte zweite Gehäuseteil 6 als wegwerfbarer Einwegartikel ausgebildet. An einem oberen Bereich 18 des zweiten Gehäuseteils 6 ist ein Anschlussstutzen 20 für eine Saugleitung 22 (vgl. Fig. 1a) vorgesehen, die dann beispielsweise bei der Verwendung der Vorrichtung 2 zur Unterdrucktherapie von Wunden zu einem die Wunde druckdicht verschließenden Wundverband führt und dort beispielsweise über einen Port mit dem Wundraum kommuniziert, um im Wundraum einen Unterdruck anzulegen und aufrechtzuerhalten und Wundsekrete in den Behälter 10 abzusaugen. Hierfür kommuniziert der Behälter 10 mit der Unterdruck erzeugenden Einrichtung, die in dem ersten Gehäuseteil 4 angeordnet ist. Bei dem weiteren Anschlussmittel 23 kann es sich um einen Instillations- oder Spülanschluss oder um einen Druckmessanschluss handeln.

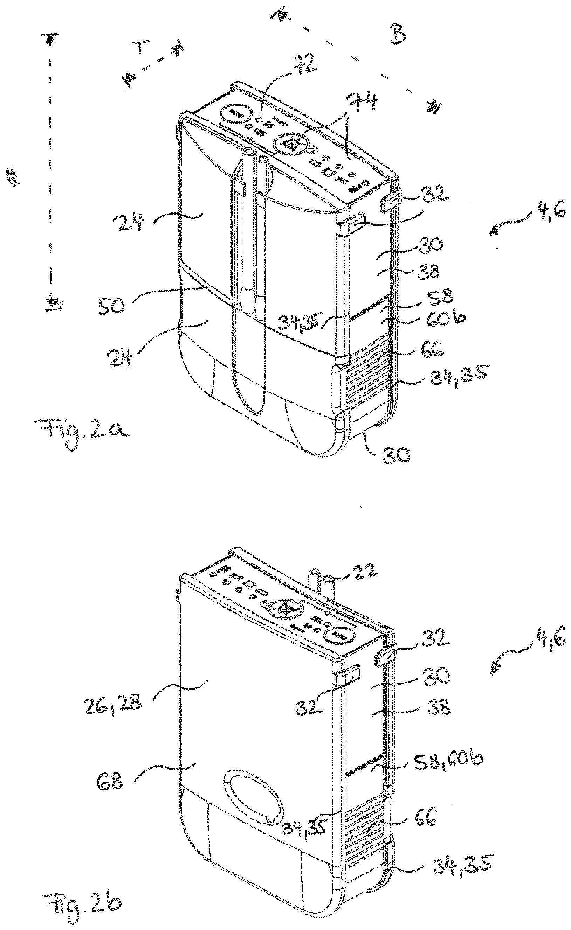

In der in den Fig. 1a bis 1c und 2a, 2b gezeigten Ausführungsform befinden sich die Gehäuseteile 4, 6 in einem bestimmungsgemäß zusammengefügten Zustand. In einer mobilen Trageposition oder Tragesituation, in der die Vorrichtung 2 beispielsweise wie eine Tragetasche über eine Schulter des Benutzers gehängt getragen wird, weist die Vorrichtung 2 eine dem Körper des Benutzers zugewandte Rückseite 24 und eine dieser gegenüberliegende vom Körper des Benutzers abgewandte und eine Sichtseite 26 der Vorrichtung bildende Vorderseite 28 auf. Zwischen der Vorder- und Rückseite 28, 24 ist eine diese verbindende Umfangsseite 30 angeordnet. Vorzugsweise ist der transparente Bereich 14 der Wandung 12 des zweiten Gehäuseteils 6 in der Umfangsseite 30 der Vorrichtung 2 angeordnet. Das gurtartige Haltemittel 8 der Vorrichtung 2 ist in einer Betriebsstellung erfindungsgemäß derart angeordnet, dass es den transparenten Bereich 14 des zweiten Gehäuseteils 6 überdeckt und eine Einsichtnahme in das Innere 16 des Behälters 10 daher verhindert ist. Gemäß der Fig. 1a umgibt das Haltemittel 8 in der Betriebsstellung die Vorrichtung 2 im Bereich der Umfangsseite 30 umfänglich zumindest teilweise, so dass die Umfangsseite 30 der Vorrichtung 2 teilweise und der transparente Bereich 14 vollständig von dem Haltemittel 8 bedeckt ist.

Des Weiteren zeigen die Figuren 1a bis 2e im Bereich der Umfangsseite 30 Führungselemente 32 für das gurtartige Haltemittel 8, insbesondere und vorzugsweise in Form von abgewinkelten Stegen und/oder Laschen, hinter welche das Haltemittel 8 gebracht und somit geführt zwischen diesen und der Umfangsseite 30 angeordnet werden kann. Es erweist sich als vorteilhaft, wenn die Umfangsseite 30 in Bezug auf einen äußeren Rand 34 einer die Vorder- oder Rückseite 28, 24 bildende Deckplatte der Vorrichtung 2 zurückversetzt angeordnet ist, so dass auf diese Weise ein Führungselement 32 in Form einer in Umfangsrichtung erstreckten Schiene oder Rippe 35 gebildet ist, welche das Haltemittel 8 entlang der Umfangsrichtung insbesondere beidseits begrenzt und führt und in der gewünschten Betriebsstellung hält.

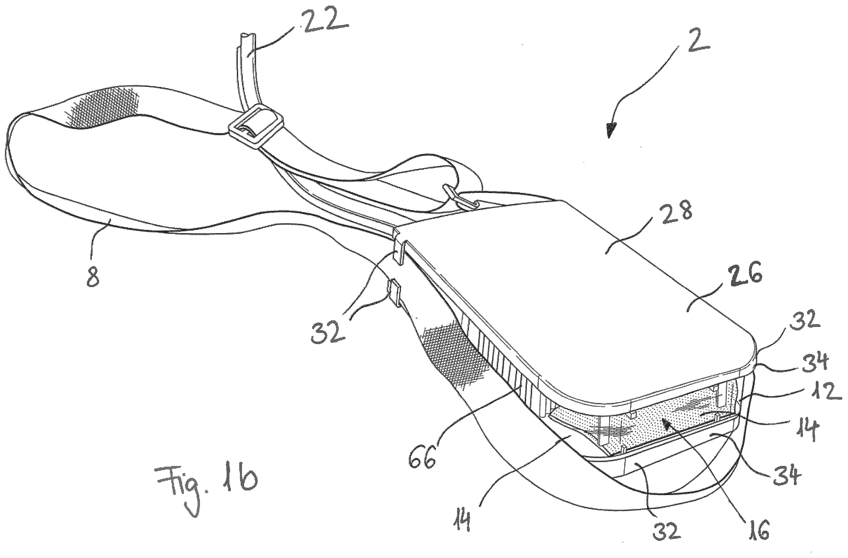

Ferner umfasst die Vorrichtung 2 Befestigungselemente 36, insbesondere Haken-/Schlaufen-Befestigungselemente oder Druckknöpfe, mit welchen das Haltemittel 8 an der Vorrichtung 2 lösbar fixierbar ist und in der Betriebsstellung gehalten werden kann, so dass das Haltemittel 8 nicht unbeabsichtigt verrutschen und einen unerwünschten Blick in das Innere 16 des Behälters 10 freigeben kann. Um eine gewollte Einsichtnahme in den Behälter 10 zu ermöglichen, kann das Haltemittel 8 von den Befestigungselementen 36 gelöst und aus der Betriebsstellung bewegt werden. Es erweist sich als vorteilhaft, wenn das Haltemittel 8 nicht komplett von den Gehäuseteilen 4, 6 abgenommen werden muss, um einen Blick in das Innere 16 des Behälters 10 freizugeben. Vorzugsweise umfasst die Vorrichtung 2 mehrere Befestigungselemente 36, die im Bereich der Umfangsseite 30, insbesondere an seitlichen Bereichen 38 und/oder an einem unteren Bereich 40 der Umfangsseite 30, angeordnet sind. In einer bevorzugten Ausführungsform kann das Haltemittel 8 an mindestens einem der Befestigungselemente 36 fixiert und um die Umfangsseite 30 herum angeordnet bleiben und nur teilweise aus der Betriebsstellung bewegt werden, um eine Einsichtnahme in den Behälter 10 zu ermöglichen (Fig. 1b).

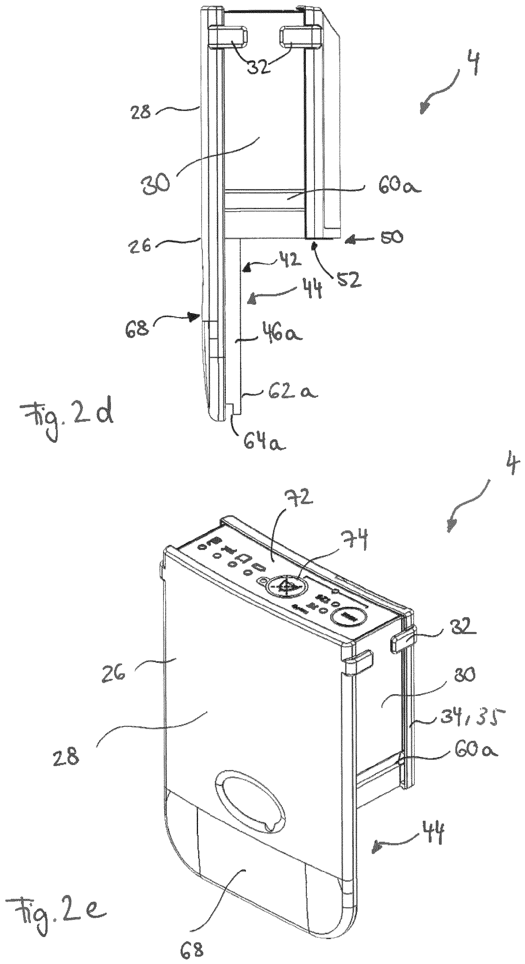

Man erkennt des Weiteren bei der dargestellten bevorzugten Ausführungsform in den Figuren 2c, 2d und 2e, dass der erste Gehäuseteil 4 in der Seitenansicht ein L-förmiges Profil mit einer Schenkelplatte 44 aufweist, deren Breite und Höhe gegenüber der Tiefe in horizontaler Richtung in der Trageposition betrachtet größer sind. Auf einer Innenseite 42 der Schenkelplatte 44 des L-förmigen ersten Gehäuseteils 4 sind beispielhaft zwei erste Schiebeführungsmittel 46a ausgebildet. Der zweite Gehäuseteil 6 umfasst zu den ersten Schiebeführungsmitteln 46a komplementäre zweite Schiebeführungsmittel 46b. Hierdurch ist der zweite Gehäuseteil 6 auf die Schenkelplatte 44 des L-förmigen ersten Gehäuseteils 4 translatorisch geführt in einer Schieberichtung 48 aufschiebbar. Die ersten Schiebeführungsmittel 46a des ersten Gehäuseteils 4 sind in der gezeigten Ausführungsform als Führungsschienen oder -rippen ausgebildet. Die zweiten Schiebeführungsmittel 46b sind als dazu komplementäre Aufnahmevertiefungen, insbesondere in Form von Schiebeführungsnuten, ausgebildet. Wenn die ersten Schiebeführungsmittel 46a in Eingriff mit den zweiten Schiebeführungsmitteln 46b sind, lässt sich der zweite Gehäuseteil 6 translatorisch auf den ersten Gehäuseteil 4 aufschieben, bis die Gehäuseteile im bestimmungsgemäß zusammengesetzten Zustand (Fig. 2a und 2b) auf Stoß aneinander anliegen und der zweite Gehäuseteil 6 das L-förmige erste Gehäuseteil 4 komplementär zu einer Art Scheibenform ergänzt. Scheibenform im Sinne der vorliegenden Erfindung bedeutet, dass die Breite B der Vorrichtung 2 in der Trageposition betrachtet in horizontaler Richtung und die Höhe H in vertikaler Richtung der zusammengefügten Gehäuseteile jeweils größer ist als die Tiefe T in horizontaler Richtung und orthogonal zur Breiten- und Höhenerstreckung. Hierdurch ist es möglich, dass die Vorrichtung 2 in Tiefenrichtung insgesamt so ausgebildet und bemessen werden kann, dass sie bequem und taschenartig am Körper eines Benutzers getragen werden kann.

Betrachtet man die Vorrichtung 2 in der Trageposition am Körper des Benutzers, so liegen die Gehäuseteile 4, 6 über eine im Wesentlichen horizontale Trennebene 50 gegeneinander an, wobei sich die Schenkelplatte 44 des ersten Gehäuseteils 4 plattenartig vorspringend über eine Erstreckung des ersten Gehäuseteils 4 hinaus fortsetzt, um ein Führungsmittel zum Fügen der beiden Gehäuseteile zu bilden.

Dessen ungeachtet befindet sich aber der die Unterdruck erzeugende Einrichtung umfassende Bereich des ersten Gehäuseteils 4 oberhalb der Trennebene 50 und der zweite Gehäuseteil 6 unterhalb der Trennebene 50.

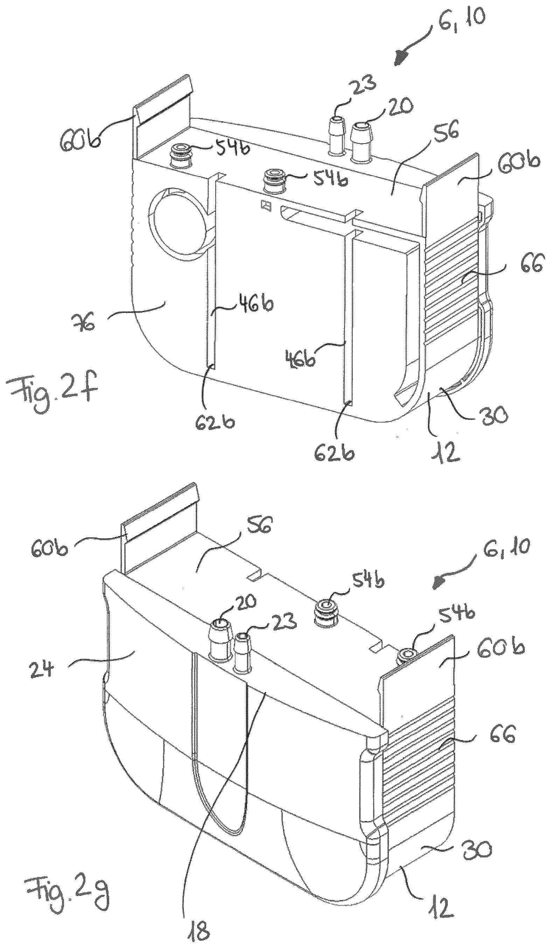

Aus der Figur 2c, welche den ersten Gehäuseteil 4 separat von dem zweiten Gehäuseteil 6 zeigt, ist unmittelbar ersichtlich, dass auf einer dem zweiten Gehäuseteil 6 zugewandten Seite 52 des ersten Gehäuseteils 4 mehrere Anschlussmittel 54a ausgebildet sind, die mit Anschlussmitteln 54b (Fig. 2f) des zweiten Gehäuseteils koppelbar sind, wenn die Gehäuseteile miteinander gefügt werden. Die dem ersten Gehäuseteil 4 zugewandte Seite 56 des zweiten Gehäuseteils 6 ist komplementär zu der Ausbildung der Seite 52 des ersten Gehäuseteils 4 ausgebildet, so dass die beiden Gehäuseteile 4, 6 nur in einer korrekten Weise miteinander gefügt bzw. aneinander befestigt werden können.

Des Weiteren umfasst die Vorrichtung 2 schnappende, rastende oder in sonstiger Weise hintergreifend wirkende Verriegelungsmittel 58, mittels derer der zweite Gehäuseteil 6 gegen den ersten Gehäuseteil 4 lösbar fixierbar ist. Durch translatorisches Aufschieben des zweiten Gehäuseteils 6 auf den ersten Gehäuseteil 4, insbesondere im Wesentlichen quer zu der horizontalen Trennebene 50, werden am zweiten Gehäuseteil 6 angeordnete Verriegelungslaschen 60b, insbesondere selbsttätig, ausgelenkt und rasten dann in eine die Gehäuseteile 4, 6 gegeneinander verriegelnde Stellung. Hierfür sind an dem ersten Gehäuseteil 4 zu den Verriegelungslaschen 60b komplementäre hintergreifbare Elemente 60a vorgesehen, die von den Verriegelungslaschen 60b hintergriffen werden. Wenn die Gehäuseteile 4, 6 in ihre verriegelte Stellung gebracht sind, wird vorzugsweise automatisch eine Unterdruckkommunikation zwischen dem Inneren des Behälters 10 des zweiten Gehäuseteils 6 und der Unterdruck erzeugenden Einrichtung hergestellt.

Gemäß der bevorzugten gezeigten Ausführungsform sind in Endbereichen 62a, 62b der ersten und zweiten Schiebeführungsmittel 46a, 46b des ersten und zweiten Gehäuseteils 4, 6 weitere Verriegelungsmittel 64a, 64b ausgebildet. Die Endbereiche 62a, 62b der Schiebeführungsmittel 46a, 46b sind derart korrespondierend profiliert ausgebildet, dass diese ineinander eingreifen, wenn die beiden Gehäuseteile 4, 6 auf Stoß aneinander anliegen.

Die Figuren 2a und 2b zeigen die Gehäuseteile 4, 6 im bestimmungsgemäß zusammengefügten Zustand, in einer Schrägansicht auf die Vorderseite 28 bzw. Sichtseite 26 der Vorrichtung 2 (Fig. 2b) und in einer Schrägansicht auf die Rückseite 24 der Vorrichtung 2 (Fig. 2a). Im gefügten Zustand der beiden Gehäuseteile 4 und 6 befinden sich die Verriegelungsmittel 58, 60a, 60b, 64a und 64b in einem die beiden Gehäuseteile 4, 6 formschlüssig aneinander haltenden verriegelten Zustand. Es ist denkbar, dass Betätigungsorgane, beispielsweise Taster, vorgesehen sind, welche betätigbar sind, um die Verriegelungsmittel 58, 60a, 60b, 64a und 64b zu lösen. Vorzugsweise sind die Verriegelungsmittel 58, 60a, 60b, 64a und 64b selbsttätig lösbar, wenn der zweite Gehäuseteil 6 in einer translatorischen Zugbewegung in einer Richtung entgegen der Schieberichtung 48 von dem ersten Gehäuseteil 4 weggezogen wird.

Wie aus den Figuren 2a bis 2c, sowie 2f und 2g ersichtlich sind am zweiten Gehäuseteil 6 zwei Griffflächen 66 ausgebildet. Zum Lösen des zweiten Gehäuseteils 6 vom ersten Gehäuseteil 4, kann der Benutzer das zweite Gehäuseteil beidseitig an den Griffflächen 66 greifen und den zweiten Gehäuseteil 6 durch die translatorische Zugbewegung vom ersten Gehäuseteil 4 abziehen. Indem der zweite Gehäuseteil auf der einen Seite mit Daumen und auf der anderen Seite mit den restlichen Fingern im Bereich der Griffflächen 66 gegriffen wird, kann der zweite Gehäuseteil 6 mit einer Hand vom ersten Gehäuseteils 4 abgenommen werden. Dies erweist sich als besonders vorteilhaft, da auf diese Weise ein mit Körperflüssigkeiten befüllter Behälter 10 mit nur einer Hand gelöst und in ein Entsorgungsbehältnis gegeben werden kann.

Gleichwohl sind die Gehäuseteile 4, 6 und die Verriegelungsmittel 58, 60a, 60b, 64a und 64b derart klemmschlüssig gegeneinander gehalten, dass ein unbeabsichtigtes Separieren der beiden Gehäuseteile 4, 6 voneinander verhindert ist. Ist das gurtartige Haltemittel 8 in der Betriebsposition angeordnet, so umläuft es (in den Fig. 2a bis 2g nicht dargestellt) die Umfangsseite 30 der Vorrichtung zumindest teilweise und verdeckt dabei den transparenten Bereich 14 in der Wandung 12 des zweiten Gehäuseteils 6. Das Haltemittel 8 ist über die Führungs- und/oder Befestigungselemente 32, 36 zumindest an dem ersten Gehäuseteil 4 fixiert.

In der bevorzugten gezeigten Ausführungsform wird die Sichtseite 26 der Vorrichtung 2 durch eine Außenseite 68 der Schenkelplatte 44 des L-förmigen ersten Gehäuseteils 4 gebildet, die vorzugsweise aus einem nicht transparenten Material gefertigt ist. In der mobilen Trageposition am Körper eines Benutzers ist somit ein Einblick in das Innere 16 des den Behälter 10 bildenden zweiten Gehäuseteils 6 für Dritte weitestgehend unmöglich, wenn der zweite Gehäuseteil 6 in frontaler Blickrichtung auf die körperabgewandte Sichtseite 26 vollständig von der Schenkelplatte 44 des ersten Gehäuseteils 4 in dieser Blickrichtung verdeckt ist und sich das gurtartige Haltemittel 8 in der Betriebsposition befindet. Eine Einsichtnahme ist selbst dann nicht möglich, wenn der zweite Gehäuseteil 6 zusätzlich zu dem transparenten Bereich 14 der Wandung 12 in der Umfangsseite 30 der Vorrichtung 2 weitere transparente Bereiche 76 (vgl. Fig. 2c) aufweist. Die transparenten Bereiche 14, 76 sind nur in den Figuren 2c und 1b als solche zeichnerisch dargestellt sind. Beispielsweise könnte die komplette Seite des zweiten Gehäuseteils, welche die zweiten Schiebeführungsmittel 46b umfasst, oder auch alle Seiten als transparenter Bereich 76 ausgebildet sein (vgl. Fig. 2f). Die Rückseite 24 der Vorrichtung 2 wird zum Teil durch den ersten und zum Teil durch den zweiten Gehäuseteil 4, 6 gebildet und ist vorzugsweise aus einem nicht transparenten Material gefertigt.

Der erste Gehäuseteil 4 weist eine langgestreckte kanalbildende Ausnehmung 70 für die am Anschlussstutzen 20 des zweiten Gehäuseteils 6 befestigte und zum Körper führende Saugleitung 22 und für die optionale Mess- und/oder Spülleitung auf.

In einem in der Trageposition betrachtetet oben liegenden Bereich 72 der Umfangsseite 30, welcher von dem in der Betriebsstellung befindlichen Haltemittel 8 nicht überdeckt wird, sind Bedien- und Anzeigeelemente 74 vorgesehen, die für den Benutzer gut zugänglich und einsehbar sind, wenn sich die Vorrichtung 2 in der mobilen Trageposition am Körper eines Benutzers befindet.

## Patentansprüche

1. Vorrichtung (2) zur Bereitstellung von Unterdruck zur Unterdruckbehandlung von Wunden am menschlichen Körper, mit einer Unterdruck erzeugenden Einrichtung und einem Behälter (10) zur Aufnahme von Flüssigkeiten in einem Inneren des Behälters, insbesondere von aus einer Wunde abgesaugten Wundsekreten, mit einem Anschluss (20) für eine zum Körper führende Saugleitung (22), wobei die Unterdruck erzeugende Einrichtung in einem ersten Gehäuseteil (4) der Vorrichtung (2) angeordnet ist und der Behälter (10) von einem zweiten Gehäuseteil (6) der Vorrichtung gebildet ist, wobei die Gehäuseteile (4, 6) lösbar gegeneinander fixierbar sind, und die Vorrichtung (2) ein gurtartiges Haltemittel (8) aufweist, so dass sie am Körper des Benutzers tragbar und mitführbar ist, wobei die Vorrichtung (2) eine in einer mobilen Trageposition dem Körper des Benutzers zugewandte Rückseite (24) und eine dieser gegenüberliegende vom Körper des Benutzers abgewandte und eine Sichtseite (26) der Vorrichtung (2) bildende Vorderseite (28) und eine diese verbindende Umfangsseite (30) umfasst, **dadurch gekennzeichnet, dass** das Haltemittel (8) derart an der Vorrichtung (2) lösbar fixierbar ist, dass das Haltemittel (8) in einer Betriebsstellung einen transparenten Bereich (14) einer Wandung (12) des zweiten Gehäuseteils (6) abdeckt, durch den eine Einsichtnahme in das Innere (16) des Behälters (10) möglich ist, wenn sich das Haltemittel (8) nicht in der Betriebsstellung befindet, wobei der transparente Bereich (14) der Wandung (12) des zweiten Gehäuseteils (6) in der Umfangsseite (30) der Vorrichtung (2) ausgebildet ist.

2. Vorrichtung (2) nach Anspruch 1, **dadurch gekennzeichnet, dass** das Haltemittel (8) in der Betriebsstellung die Vorrichtung (2) im Bereich der Umfangsseite (30) umfänglich zumindest teilweise umgibt, so dass die Umfangsseite (30) der Vorrichtung (2) zumindest teilweise von dem Haltemittel (8) bedeckt ist.

3. Vorrichtung (2) nach einem oder mehreren der vorhergehenden Ansprüche, **dadurch gekennzeichnet, dass** die Vorrichtung (2) wenigstens ein Führungselement (32) für das Haltemittel (8) aufweist.

4. Vorrichtung (2) nach Anspruch 3, **dadurch gekennzeichnet, dass** ein Führungselement (32) entlang der Umfangsseite (30) in Umfangsrichtung erstreckt ist.

5. Vorrichtung nach Anspruch 4, **dadurch gekennzeichnet, dass** das Führungselement (32) durch eine über die Umfangsseite vorspringende die Vorder- und/oder Rückseite (28, 24) bildende Deckplatte gebildet ist.

6. Vorrichtung (2) nach einem oder mehreren der vorhergehenden Ansprüche, **dadurch gekennzeichnet, dass** die Vorrichtung (2) Befestigungselemente (36), insbesondere Haken-/Schlaufen-Befestigungselemente, aufweist, an denen das Haltemittel (8) lösbar fixierbar ist.

7. Vorrichtung (2) nach einem oder mehreren der vorhergehenden Ansprüche, **dadurch gekennzeichnet, dass** die Gehäuseteile (4, 6) über eine Schiebesitzführung aneinander gefügt werden.

8. Vorrichtung (2) nach einem oder mehreren der vorhergehenden Ansprüche, **dadurch gekennzeichnet, dass** wenigstens ein Gehäuseteil (4, 6) in einer Seitenansicht im Wesentlichen L-förmig ausgebildet oder ergänzt ist, wobei die L-Form eine Schiebesitzführung bildet.

9. Vorrichtung (2) nach Anspruch 8, **dadurch gekennzeichnet, dass** auf einer Innenseite (42) einer Schenkelplatte (44) des L-förmigen Gehäuseteils (4, 6) wenigstens ein erstes Schiebeführungsmittel (46a) ausgebildet ist, und der andere Gehäuseteil (4, 6) ein zu dem ersten Schiebeführungsmittel (46a) komplementäres zweites Schiebeführungsmittel (46b) umfasst, so dass der andere Gehäuseteil (4, 6) auf die Schenkelplatte (44) des L-förmigen Gehäuseteils (4, 6) translatorisch geführt aufschiebbar ist, wobei die Schiebeführungsmittel (46a, 46b) ineinander eingreifen.

10. Vorrichtung (2) nach einem oder mehreren der vorhergehenden Ansprüche 7 bis 9, **dadurch gekennzeichnet, dass** die Schiebesitzführung und/oder das erste und/oder das zweite Schiebeführungsmittel (46a, 46b) wenigstens eine Führungsschiene, -rippe, - steg und/oder wenigstens eine dazu komplementäre Führungsnut, -aufnahme, -vertiefung umfassen.

11. Vorrichtung (2) nach einem oder mehreren der vorhergehenden Ansprüche, **dadurch gekennzeichnet, dass** der erste Gehäuseteil (4) in der Seitenansicht im Wesentlichen L-förmig ausgebildet ist und der zweite Gehäuseteil (6) ein den ersten Gehäuseteil (4) komplementär ergänzendes Profil aufweist, so dass die Vorrichtung (2) im Wesentlichen eine Scheibenform aufweist, wenn sich die Gehäuseteile (4, 6) in dem bestimmungsgemäß gefügten Zustand befinden.

12. Vorrichtung (2) nach einem oder mehreren der vorhergehenden Ansprüche, **dadurch gekennzeichnet, dass** die Sichtseite (26) der Vorrichtung zumindest teilweise durch eine Schenkelplatte (44) eines L-förmigen Gehäuseteils (4, 6) und die Rückseite zumindest teilweise durch eine hintere Wand des anderen Gehäuseteils (4, 6) gebildet wird.

13. Vorrichtung (2) nach einem oder mehreren der vorhergehenden Ansprüche, **dadurch gekennzeichnet, dass** die Sichtseite (26) und/oder die Rückseite (24) der Vorrichtung (2) aus einem nicht transparenten Material gefertigt ist, und im bestimmungsgemäß zusammengefügten Zustand eine Schenkelplatte (44) des ersten Gehäuseteils (4) den zweiten Gehäuseteil (6) in frontaler Blickrichtung auf die körperabgewandte Sichtseite (26) der Vorrichtung (2) zu wenigstens 90% oder vorzugsweise vollständig verdeckt.

14. Vorrichtung (2) nach einem oder mehreren der vorhergehenden Ansprüche, **dadurch gekennzeichnet, dass** der erste und der zweite Gehäuseteil (4, 6) der Vorrichtung (2) im bestimmungsgemäß zusammengesetzten Zustand in der mobilen Trageposition betrachtet vertikal übereinander angeordnet sind, so dass zwischen den Gehäuseteilen (4, 6) eine im Wesentlichen horizontale Trennebene (50) ausgebildet ist, wobei ein oberer Teil der Vorrichtung (2) von dem ersten, die Unterdruck erzeugende Einrichtung umfassenden Gehäuseteil (4) und ein unterer Teil der Vorrichtung von dem zweiten den Behälter bildenden Gehäuseteil (6) gebildet wird.

15. Vorrichtung (2) nach einem oder mehreren der vorhergehenden Ansprüche, **dadurch gekennzeichnet, dass** die Vorrichtung (2) in einem in der Trageposition oberen Teil (72) der Umfangsseite (30), welcher von dem in der Betriebsstellung befindlichen Haltemittel (8) nicht überdeckt wird, Bedien- und Anzeigeelemente (74) aufweist.

## Claims

1. Device (2) for providing a vacuum for vacuum-treating wounds on the human body, comprising a vacuum-generating apparatus and a container (10) for receiving liquids in an interior of the container, in particular wound secretions suctioned from a wound, said container comprising a connection (20) for a suction line (22) leading to the body, the vacuum-generating apparatus being arranged in a first housing part (4) of the device (2) and the container (10) being formed by a second housing part (6) of the device, it being possible for the housing parts (4, 6) to be releasably fixed to one another, and the device (2) having a beltlike holding means (8) such that it can be worn on the user's body and carried around, the device (2) having a rear face (24), which faces the user's body in a mobile wearing position, a front face (28), which is opposite said rear face, faces away from the user's body and forms a visible face (26) of the device (2), and a peripheral face (30), which connects said front face and said rear face, **characterized in that** the holding means (8) can be releasably fixed to the device (2) in such a way that the holding means (8) cover a transparent region (14) of a wall (12) of the second housing part (6) in an operating position, through which transparent region the interior (16) of the container (10) can be inspected when the holding means (8) is not in the operating position, the transparent region (14) of the wall (12) of the second housing part (6) being formed in the peripheral face (30) of the device (2).

2. Device (2) according to claim 1, **characterized in that**, in the operating position, the holding means (8) at least partially surrounds the periphery of the device (2) in the region of the peripheral face (30) such that the peripheral face (30) of the device (2) is at least partially covered by the holding means (8).

3. Device (2) according to one or more of the preceding claims, **characterized in that** the device (2) has at least one guide element (32) for the holding means (8).

4. Device (2) according to claim 3, **characterized in that** a guide element (32) extends along the peripheral face (30) in the peripheral direction.

5. Device according to claim 4, **characterized in that** the guide element (32) is formed by a cover plate which projects beyond the peripheral face and forms the front and/or rear face (28, 24).

6. Device (2) according to one or more of the preceding claims, **characterized in that** the device (2) has fastening elements (36), in particular hook-and-loop fastening elements, to which the holding means (8) can be releasably fixed.

7. Device (2) according to one or more of the preceding claims, **characterized in that** the housing parts (4, 6) are joined to one another via a sliding seat guide.

8. Device (2) according to one or more of the preceding claims, **characterized in that**, in a side view, at least one housing part (4, 6) is substantially in an L shape or is added to such that an L shape is substantially formed, the L shape forming a sliding seat guide.

9. Device (2) according to claim 8, **characterized in that** at least one first sliding guide means (46a) is formed on an inner face (42) of a leg plate (44) of the L-shaped housing part (4, 6), and the other housing part (4, 6) comprises a second sliding guide means (46b) which is complementary to the first sliding guide means (46a) such that the other housing part (4, 6) can be pushed onto the leg plate (44) of the L-shaped housing part (4, 6) in a translationally guided manner, the sliding guide means (46a, 46b) engaging in one another.

10. Device (2) according to one or more of the preceding claims 7 to 9, **characterized in that** the sliding seat guide and/or the first and/or the second sliding guide means (46a, 46b) have at least one guide rail, rib or projection and/or at least one guide groove, receptacle or recess that is complementary to said guide rail, rib or projection.

11. Device (2) according to one or more of the preceding claims, **characterized in that** the first housing part (4) is substantially L-shaped in the side view and the second housing part (6) has a profile that is added to the first housing part (4) in a complementary manner such that the device (2) is substantially disk-shaped when the housing parts (4, 6) are joined as intended.

12. Device (2) according to one or more of the preceding claims, **characterized in that** the visible face (26) of the device is formed at least in part by a leg plate (44) of an L-shaped housing part (4, 6) and the rear face is formed at least in part by a rear wall of the other housing part (4, 6).

13. Device (2) according to one or more of the preceding claims, **characterized in that** the visible face (26) and/or the rear face (24) of the device (2) is made of a non-transparent material and, when the housing parts are joined as intended, a leg plate (44) of the first housing part (4) covers at least 90% or preferably all of the second housing part (6) in a viewing direction from the front onto the visible face (26) of the device (2) that faces away from the body.

14. Device (2) according to one or more of the preceding claims, **characterized in that**, when assembled as intended, the first and the second housing parts (4, 6) of the device (2) are arranged vertically one above the other when viewed in the mobile wearing position such that a substantially horizontal separating plane (50) is formed between the housing parts (4, 6), an upper part of the device (2) being formed by the first housing part (4) comprising the vacuum-generating apparatus and a lower part of the device being formed by the second housing part (6) which forms the container.

15. Device (2) according to one or more of the preceding claims, **characterized in that** the device (2) comprises operating and display elements (74) in a part (72) of the peripheral face (30) which is at the top in the wearing position, and which is not covered by the holding means (8) in the operating position.

## Revendications

1. Dispositif (2) destiné à fournir de la pression négative pour le traitement par pression négative de plaies sur le corps humain, avec un dispositif générateur de pression négative et un récipient (10) destiné à recevoir des liquides dans un intérieur du récipient, en particulier des sécrétions de plaie aspirées au niveau d'une plaie, avec un raccord (16) pour une conduite d'aspiration (22) menant vers le corps, dans lequel le dispositif générateur de pression négative est agencé dans une première partie de boîtier (4) du dispositif (2) et le récipient (10) est formé par une deuxième partie de boîtier (6) du dispositif, dans lequel les parties de boîtier (4, 6) peuvent être fixées de manière amovible l'une contre l'autre, et le dispositif (2) présente un moyen de maintien (8) de type ceinture de sorte qu'il puisse être porté sur le corps de l'utilisateur et que l'on puisse l'avoir avec soi, dans lequel le dispositif (2) comprend une face arrière (24) qui est tournée vers le corps de l'utilisateur dans une position de port mobile, et une face avant (28) qui est située en regard de cette dernière, montre dans la direction opposée au corps de l'utilisateur et forme une face visible (26) du dispositif (2), ainsi qu'une face périphérique (30) qui relie celles-ci, **caractérisé par le fait que** le moyen de maintien (8) peut être fixé de manière amovible au dispositif (2) de telle sorte que le moyen de maintien (8) recouvre dans une position de fonctionnement une zone transparente (14) d'une paroi (12) de la deuxième partie de boîtier (6), qui permet de voir dans l'intérieur (16) du récipient (10) lorsque le moyen de maintien (8) ne se trouve pas dans la position de fonctionnement, dans lequel la zone transparente (14) de la paroi (12) de la deuxième partie de boîtier (6) est réalisée dans la face périphérique (30) du dispositif (2).

2. Dispositif (2) selon la revendication 1, **caractérisé par le fait que**, en position de fonctionnement, le moyen de maintien (8) entoure circonférentiellement au moins en partie le dispositif (2) au niveau de la face périphérique (30) de sorte que la face périphérique (30) du dispositif (2) est recouverte au moins en partie par le moyen de maintien (8).

3. Dispositif (2) selon une ou plusieurs des revendications précédentes, **caractérisé par le fait que** le dispositif (2) comprend au moins un élément de guidage (32) pour le moyen de maintien (8).

4. Dispositif (2) selon la revendication 3, **caractérisé par le fait qu'**un élément de guidage (32) s'étend le long de la face périphérique (30) dans le sens circonférentiel.

5. Dispositif selon la revendication 4, **caractérisé par le fait que** l'élément de guidage (32) est formé par une plaque de recouvrement qui fait saillie de la face circonférentielle et forme la face avant et/ou la face arrière (28, 24).

6. Dispositif (2) selon une ou plusieurs des revendications précédentes, **caractérisé par le fait que** le dispositif (2) comprend des éléments de fixation (36), en particulier des éléments de fixation à crochets et boucles, sur lesquels le moyen de maintien (8) peut être fixé de manière amovible.

7. Dispositif (2) selon une ou plusieurs des revendications précédentes, **caractérisé par le fait que** les parties de boîtier (4, 6) sont jointes l'une à l'autre par l'intermédiaire d'un guide de siège coulissant.

8. Dispositif (2) selon une ou plusieurs des revendications précédentes, **caractérisé par le fait que**, vue de côté, au moins une partie de boîtier (4, 6) est réalisée ou complétée pour l'essentiel en forme de L, dans lequel la forme en L forme un guide de siège coulissant.

9. Dispositif (2) selon la revendication 8, **caractérisé par le fait qu'**au moins un premier moyen de guidage coulissant (46a) est formé sur une face intérieure (42) d'une plaque de branche (44) de la partie de boîtier (4, 6) en forme de L, et que l'autre partie de boîtier (4, 6) comprend un deuxième moyen de guidage coulissant (46b) qui est complémentaire du premier moyen de guidage coulissant (46a) de sorte que l'autre partie de boîtier (4, 6) peut être poussée sur la plaque de branche (44) de la partie de boîtier (4, 6) en forme de L en étant guidée en translation, dans lequel les moyens de guidage coulissant (46a, 46b) s'engagent les uns dans les autres.

10. Dispositif (2) selon une ou plusieurs des revendications 7 à 9 précédentes, **caractérisé par le fait que** le guide de siège coulissant et/ou le premier et/ou le deuxième moyen de guidage coulissant (46a, 46b) comprennent au moins un rail, nervure, entretoise de guidage et/ou au moins une rainure, logement, creux de guidage qui est complémentaire de celui-ci/celle-ci,

11. Dispositif (2) selon une ou plusieurs des revendications précédentes, **caractérisé par le fait que**, vue de côté, la première partie de boîtier (4) est réalisée pour l'essentiel en forme de L et que la deuxième partie de boîtier (6) présente un profil qui complète de manière complémentaire la première partie de boîtier (4) de sorte que le dispositif (2) présente pour l'essentiel une forme de disque lorsque les parties de boîtier (4, 6) sont à l'état assemblé comme prévu.

12. Dispositif (2) selon une ou plusieurs des revendications précédentes, **caractérisé par le fait que** la face visible (26) du dispositif est formée au moins en partie par une plaque de branche (44) d'une partie de boîtier (4, 6) en forme de L et que la face arrière est formée au moins en partie par une paroi arrière de l'autre partie de boîtier (4, 6).

13. Dispositif (2) selon une ou plusieurs des revendications précédentes, **caractérisé par le fait que** la face visible (26) et/ou la face arrière (24) du dispositif (2) est réalisée à partir d'un matériau non transparent, et que, à l'état assemblé comme prévu, une plaque de branche (44) de la première partie de boîtier (4) recouvre la deuxième partie de boîtier (6) à au moins 90 % ou de préférence complètement dans la direction de regard frontale sur la face visible (26) du dispositif (2), laquelle montre dans la direction opposée au corps.

14. Dispositif (2) selon une ou plusieurs des revendications précédentes, **caractérisé par le fait que**, à l'état assemblé comme prévu, vues en position de port mobile, la première et la deuxième partie de boîtier (4, 6) du dispositif (2) sont disposées verticalement l'une au-dessus de l'autre de sorte qu'un plan de séparation (50) pour l'essentiel horizontal est formé entre les parties de boîtier (4, 6), dans lequel une partie supérieure du dispositif (2) est formée par la première partie de boîtier (4) comprenant le dispositif générateur de pression négative et une partie inférieure du dispositif est formée par la deuxième partie de boîtier (6) formant le récipient.

15. Dispositif (2) selon une ou plusieurs des revendications précédentes, **caractérisé par le fait que** le dispositif (2) présente des éléments de commande et d'affichage (74) dans une partie (72) de la face périphérique (30), qui est située en haut dans la position de port et qui n'est pas recouverte par le moyen de maintien (8) se trouvant en position de fonctionnement.
